# EUROPÄISCHE PATENTSCHRIFT

(11) **EP 3 636 207 B1**
(45) Veröffentlichungstag und Bekanntmachung des Hinweises auf die Patenterteilung: **29.12.2021**
(21) Anmeldenummer: 19191752.5
(22) Anmeldetag: 14.08.2019
(51) Int. Cl.: A61C 7/36, A61F 5/56, A61L 27/18, A63B 71/08, B33Y 70/10, H04R 1/10, B29C 64/106

(54) **MATERIAL FÜR DIE ADDITIVE HERSTELLUNG VON DREIDIMENSIONALEN OBJEKTEN SOWIE VERFAHREN ZUR HERSTELLUNG UND VERWENDUNG**
MATERIAL FOR THE ADDITIVE PRODUCTION OF THREE-DIMENSIONAL OBJECTS AND METHOD FOR THE PRODUCTION AND USE THEREOF
MATÉRIAU POUR LA FABRICATION ADDITIVE D'OBJETS TRIDIMENSIONNELS AINSI QUE PROCÉDÉ DE FABRICATION ET UTILISATION

(30) Priorität: 11.10.2018 DE 102018125177
(43) Veröffentlichungstag der Anmeldung: 15.04.2020
(73) Patentinhaber: Dreve ProDiMed GmbH, 59423 Unna (DE)
(72) Erfinder: Veit, Thomas,, 48151 Münster (DE); Civelek, Emre, 59192 Bergkamen (DE)
(74) Vertreter: Köchling, Conrad-Joachim

(56) Entgegenhaltungen:
- EP-A1- 2 676 633
- EP-A1- 3 222 689
- WO-A1-2018/003381
- DE-A1-102005 050 185
- DATABASE WPI Week 201618 Thomson Scientific, London, GB; AN 2015-70928H XP002797707, & CN 104 914 675 A (LEDAO STRATEGIC MATERIALS CO LTD) 16. September 2015 (2015-09-16)
- DATABASE WPI Week 201641 Thomson Scientific, London, GB; AN 2016-086728 XP002797710, & CN 105 278 239 A (UNIV QINGDAO SCI & TECHNOLOGY) 27. Januar 2016 (2016-01-27)

## Beschreibung

Die Erfindung betrifft ein elastomeres Material für die additive Herstellung von dreidimensionalen Objekten sowie Verfahren zur Herstellung und Verwendung.

Unter "additiver Herstellung" ist die sogenannte "stereolithographische Herstellung", nämlich die Herstellung mit Digital Light Processing-Verfahren zu verstehen.

Bei herkömmlichen elastischen Materialien für die additive Herstellung dreidimensionaler Objekte, bei der Schicht für Schicht aufgebaut wird, werden silikonähnliche, elastische Materialien beschrieben, welche einen geringen oder keinen Anteil an SiloxanGruppen enthalten, keine Biokompabilität aufweisen, nicht transparent sind und/oder nicht mit dem bekannten DLP-Verfahren (DLP = Digital Light Processing) gedruckt werden können. Die Schichtbildung während des DLP-Verfahrens erfolgt durch Verfestigung der flüssigen Komponente aufgrund der Bestrahlung mit einer Lichtquelle, insbesondere mit einer Wellenlänge zwischen 355 nm und 410 nm.

Zwei Techniken des 3D-Drucks sind bekannt. Die als erstes beschriebene Technik ist von Hull aus dem Jahr 1984 mit der US-Patent Nr. 5.236.637. Weitere bekannte Beispiele sind beschrieben in US-Patent Nr. 7.438.846, US-Patent Nr. 7.982.474 von M. Joyce, US-Patent App. 2013/0292862 von Y. Pan et al., US-Patent App. 2013/0295212 von Y. Pan et al. und zahlreiche weitere Referenzen. Materialien für die Verarbeitung mit dem DLP-Verfahren sind in der Regel begrenzt, und es besteht Bedarf an neuen Harzen, die unterschiedliche Materialeigenschaften für verschiedene Produktgruppen bieten, um das Potential der dreidimensionalen Fertigung vollständig zu nutzen.

Aus der CN 104914675 A ist eine niedriviskose, lichthärtbare Silikonformulierung für 3- D- Laser-Rapid- Prototyping, stereolithographie bzw. UV- 3-D-Druck bekannt.

Die Formulierungen enthalten 0,1- 30 Gew.-% eines niedrig-viskosen Methacrylat- Silikons. U.a. 2-30 Gew.-% eines photohärtbaren Verbindung mit ungesättigtenr funktioneller Gruppe, 1-20 Gew.-% eines Füllstoffes, 2-20 Gew.-% Photoinitiatoren und 0,1-5 Gew.-% Hilfsstoffe. Es handelt sich bei dem niedrig-viskosen Methacrylat-Silikon um zweifach Methacrylat- funktionalisierte Siloxane, wobei die bevorzugten photohärtbaren Verbindungen mit ungesättigter funktioneller Gruppe Acrylate sind, einschließlich Methyl-Isobornylacrylat und als Füllstoff u.a. feine gefällte Kieselsäure genannt ist.

Die Formulierungen von WO2018/003381 A1 enthalten 100 Teile eines zweifach (Meth)acrylat-funktionalisierten Siloxans, 1 bis 500 Teile (pro 100 Teile des Siloxans) einer siloxanfreien (Meth)acrylatverbindung, 0,1 bis 20 Teile eines Photoinitiators sowie übliche Hilfsstoffe. Die bevorzugten siloxanfreien ((Meth)acrylatverbindungen sind Isobornylacrylat und die Viskosität der Formulierungen liegt unter 2 Pa·s, bevorzugt unter 1 Pa·s. Die Viskositäten der beispielhaften zweifach (Meth)acrylat- funktionalisierten Siloxane liegt bei 0,0800- 0,110 Pa·s.

Aus der EP 2 676 633 A1 ist eine Formulierung bekannt, die ein Silikon, Urethan, Methacrylat oder ein Gemisch aus mindestens zwei dieser Polymere umfasst. Dabei handelt es sich um strahlenhärtbare Silikone, bei denen ein Katalysator zur UV-Vernetzung verwendet wird. Hierbei können auch Silikonöle als Verdünner eingesetzt werden und verstärkende Füllstoffe.

Ausgehend vom Stand der Technik liegt der Erfindung die Aufgabe zugrunde, hinsichtlich niedriger Viskosität und Elastizität verbesserte, lichthärtbarere 1K- Silikonformulierungen bereitzustellen, die in gehärtetem Zustand transparent und biokompatibel sind.

Die Lösung der Aufgabe ist in den Patentansprüchen angegeben.

Bei der Erfindung handelt es sich um ein strahlungshärtendes, niedrigviskoses, transparentes und biokompatibles 1K Silikonharz, welches zur Herstellung von elastischen dreidimensionalen Medizinprodukten und medizintechnischen Formteilen mittels stereolithographischer Verfahren, nämlich Digital Light Processing-Verfahren dient. . Dabei stehen insbesondere im otologischen Bereich Ohrpassstücke (Otoplastiken) und dentale Applikationen wie zum Beispiel Zahnfleischmasken und Mundschutz sowie kieferorthopädische Anwendungen wie Positioner und Schienen (z. B. Schnarchschienen, Aligner-Schienen) im Fokus. Die genannten Anwendungen sind als Beispiele und nicht als ausschließlich zu betrachten. Es besteht vorzugsweise aus mindestens 50 % Siloxan Gruppen, einem Siloxan Copolymer oder mindestens einem Bestandteil, welcher die Gruppe der Siloxane umfasst. Enthaltend sind: a) 25-75 Gew.-% eines monomeren oder oligomeren Dimethacrylats auf Basis von Siloxanurethanen mit einer Viskosität von < 100 Pa·s; b) 20-50 Gew.-% eines oder mehrerer monomer/oligomer cyclischer (Meth)acrylate mit einer Viskosität von < 0,5 Pa·s; gemäß Anspruch 3 bis zu 20 Gew.-% eines aliphatischen oder cycloaliphatischen Dimethacrylats mit einer Viskosität von < 10 Pa·s; c) 1-25 Gew.-% Silikonöl mit einer Viskosität von < 1 Pa·s; d) 0,5-20 Gew.-% Füllstoffe mit einer Partikelgröße < 100 µm; e) 0,1-5 Gew.-% eines oder einer Kombination mehrerer Photoinitiatoren, deren Absorption im Wellenlängenbereich des eingesetzten Laserstrahles bzw. der eingesetzten Bestrahlungsquelle liegt; gemäß Anspruch 6 bis zu 0,5 Gew.-% eines oder mehrerer anaerober Inhibitoren; gemäß Anspruch 7 bis zu 5 Gew.-% an Farbmitteln; gemäß Anspruch 8 bis zu 5 Gew.-% an üblichen Additiven wie UV-Stabilisatoren, Haftvermittlern und Verlaufsadditiven, wobei der Anteil aller Komponenten zusammen 100 Gew.-% ergibt.

Die in Gew.-% aufgezählten Angaben der einzelnen Komponenten und die unterschiedlichen Varianten der Photoinitiatoren und Additive werden in den Ansprüchen detailliert beschrieben. Neu und erfinderisch an dieser Zusammensetzung ist insbesondere, dass diese mindestens 50 % Siloxan Gruppen enthält, welche endständig (Meth)acrylat funktionalisiert sind und gleichzeitig die Eigenschaften Transparenz, Elastizität, Biokompatibilität, additiv herstellbar in einem 1K-Material zusammenfasst. Wie zum Stand der Technik angegeben, gibt es ähnliche Materialien, jedoch fasst keines dieser Materialien die aufgezählten Eigenschaften in einem Material zusammen. Der Vorteil der endfunktionalisierten Siloxan(meth)acrylate gegenüber anderen endständig (Meth)acrylat funktionalisierten Gruppen ist, dass sie physiologisch als verträglich gelten, weshalb sie in der Medizinproduktebranche häufig zum Einsatz kommen. Endständige (Meth)acrylate können sein: aliphatische oder cycloaliphatische Urethan(meth)acrylate, aliphatische oder cycloaliphatische (Meth)acrylate und n-fach ethoxylierte Bisphenol-A-Di(meth)acrylate.

Bei der Erfindung handelt es sich um ein mittels additiver Verfahren, nämlich Digital Light Processing, druckbares, transparentes, elastisches und biokompatibles 1K Silikon für die generative Fertigung.

Die Erfindung betrifft Materialien, welche durch das stereolithographische Verfahren "Digital Light Processing" (DLP) für die Herstellung von festen, transparenten, elastischen, biokompatiblen und dreidimensionalen Ohrpassstücken (Otoplastiken, Zahnfleischmasken und Mundschutz sowie kieferorthopädischen Anwendungen wie Positionern und Schienen(z. B. Schnarchschienen, Aligner-Schienen) aus niedrigviskosen 1K Materialien mit einem Siloxananteil von mindestens 50 % erhalten werden.

Es ist wichtig zu erwähnen, dass das Material zur Herstellung von elastischen, transparenten, biokompatiblen und dreidimensionalen Ohrpassstücken (Otoplastiken),Zahnfleischmasken und Mundschutz sowie kieferorthopädischen Anwendungen wie Positionern und Schienen(z. B. Schnarchschienen, Aligner-Schienen) mit einem Mindestanteil von 50 % Siloxan Gruppen erfinderisch und neu ist.

In der vorliegenden Erfindung wird ein transparentes, elastisches und biokompatibles 1K Material gemäß den Ansprüchen zur Herstellung eines dreidimensionalen Ohrpassstücks (Otoplastik),einer Zahnfleischmaske oder eines Mundschutz sowie kieferorthopädischen Anwendungen wie Positionern und Schienen(z. B. Schnarchschienen, Aligner-Schienen)mit einem Mindestanteil von 50 % an Siloxan Gruppen dargestellt, das durch folgende Eigenschaften zu beschreiben ist:
Die Erfindung stellt eine unter (UV-)Licht in wenigen Sekunden härtende Siloxanzusammensetzung zur Verfügung, welche zur Herstellung von transparenten, elastischen, biokompatiblen und drei dimensionalen Ohrpassstücken (Otoplastiken)oder Zahnfleischmasken oder eines Mundschutzes sowie kieferorthopädischer Anwendungen wie Positionern und Schienen(z. B. Schnarchschienen, Aligner-Schienen)genutzt werden kann. Insbesondere werden die zur Verfügung gestellten Zusammensetzungen gemäß den Ansprüchen hergestellt, wobei neben cyclischen (Meth)acrylaten auch aliphatische oder cycloaliphatische Di(meth)acrylate, Silikonöle und Füllstoffe eingesetzt werden. Durch den Einsatz von einem oder der Kombination aus mehreren Photoiniatoren können durch die vorhandenen (Meth)acrylate schnelle und präzise Aushärtungen der Zusammensetzung ermöglicht werden.

### Kurze Beschreibung der verwandten Materialien:

Schnellhärtende Zusammensetzungen, die sowohl einen hohen Siloxananteil besitzen, elastische Eigenschaften aufweisen, transparent und biokompatibel als auch durch additive Verfahren herstellbar sind, sind bisher nicht vorhanden. Vereinzelt können diese Eigenschaften in einigen Produkten wiedergefunden werden, aber nicht vollständig in dieser Kombination. Daher werden diese Produkte nachfolgend der Erfindung gegenüber gestellt. Die Herstellung von Silikonelastomeren durch ein generatives Verfahren zur Herstellung von Formteilen wurde von der Wacker Chemie AG in Patent App. WO2016071241 beschrieben. In dieser Anmeldung wurden keine neuen Materialien, sondern ein neues Verfahren zur Herstellung von Silikonen beschrieben. Da es sich bei dieser Methode um ein 2K-Material handelt und innerhalb des Prozesses Tropfen gelegt werden, welche anschließend strahlengehärtet werden, kann dieses Silikon nicht mit der vorliegenden Erfindung verglichen werden. Zum einen können mit dem Digital Light Processing Verfahren Genauigkeiten von 20 µm bis 100 µm (Standard: 100 µm) pro Schichtdicke erreicht werden, welche für Biomedizinprodukte wie Ohrpassstücke oder Zahnfleischmasken oder Mundschutze sowie kieferorthopädische Anwendungen wie Positioner und Schienen(z. B. Schnarchschienen, Aligner-Schienen)essentiell sind. Zum anderen härtet das 2K-Silikon von Wacker AG nach dem Mischen auch ohne Bestrahlung, was bei einem Ein Komponenten Material nicht der Fall ist. Der Grund dafür liegt in dem chemischen Mechanismus der Reaktionen. Während die Polymerisation des 2K Silikon von Wacker AG mit einem Katalysator nach dem Mischen der zwei Komponenten aktiviert wird und durch (UV-)Licht beschleunigt wird, benötigt der in dieser Erfindung genutzte bzw. die Kombination der genutzten Photoninitiatoren (UV-)Licht zum Aktivieren der Polymerisation. Dadurch wird eine längere Topfzeit gewährleistet. Das von Carbon Inc. beschriebene "Blockierte dual härtende Silikon Harze für die additive Fertigung" mit der WO2017112751, welches keine hohen Gew.-% an Siloxan Gruppen aufweist, kann nicht durch standardisierte Stereolithographie wie das DLP-Verfahren gedruckt werden und ist nicht transparent. Zudem beschreibt Carbon Inc. ein Zwei Komponenten Harz, welches zunächst mittels (UV-)Licht gehärtet wird und anschließend mit Hitze verarbeitet werden muss, um die finalen Eigenschaften, welche in der zuvor erwähnten Patentanmeldung beschrieben wurden, aufweisen zu können. Das in dieser Erfindung beschriebene Material benötigt keine anschließende Verarbeitung mit Hitze, um mechanisch belastbare Eigenschaften aufweisen zu können. (UV-)Licht härtbare Siloxane werden unter anderem auch von Shin Etsu Chemical Co. in der EP3222689 beschrieben. Die mechanischen Eigenschaften und Belastbarkeiten der hergestellten Silikone sind dabei weitaus unter dem Standard, um damit stabile Formkörper mittels Stereolithographie, insbesondere dem DLP-Verfahren zu erstellen. Auch in Patentanmeldung WO2017155919 werden lichthärtende Silikone von der Dow Corning Corporation beschrieben. Auch diese Silikone sind nicht für die Herstellung mittels stereolithographischer Verfahren, insbesondere dem DLP-Verfahren geeignet.

Der gesamte Prozess, begonnen vom dreidimensionalen Druck, bei dem Schicht für Schicht aufgebaut wird, bis hin zur Nachreinigung und Nachhärtung der gebauten Objekte, erfolgt analog zu den der bekannten Harze für den 3D-Druck wie im Artikel "Digitale Fertigung in der Dentaltechnologie mittels additiver Fertigungsverfahren" von C. Glodecki, A. Neumeister, Jahrbuch Digitale Dental Technologien 2017, S. 97-105 erläutert. Diese Übertragbarkeit und Kompatibilität konnte durch die Erfinder durch die Auswahl der richtigen Komponenten und Veränderung der Mengenverhältnisse innerhalb der Zusammensetzung explizit für das DLP-Verfahren eingestellt und optimiert werden. Insbesondere die Fließfähigkeit ist ausschlaggebend für den Bauprozess, die Nachreinigung und die erhaltenen Eigenschaften sowie die mechanische Belastbarkeit. Zudem kommen die Transparenz, Biokompabilität und die einfache Handhabung hinzu, welche nicht vernachlässigt wurden.

Als Erklärung der leichten Übertragbarkeit des neuen Materials auf das DLP-Verfahren sind die (Meth)acrylat-Funktionalitäten zu nennen, welche radikalisch polymerisiert werden und in den kommerziell für das DLP-Verfahren erhältlichen Harzen ebenfalls enthalten sind. Das von Wacker Chemie AG erwähnte Patent berichtet über reine zwei Komponenten Silikone, welche ein durch UV-Licht beschleunigtes Katalysatorsystem nutzen, um anschließend die additionsvernetzende Reaktion nach dem bekannten Mechanismus der Hydrosilylierung durchzuführen. Diese Materialien sind aufgrund ihrer hohen Reaktivitätsübertragung auf das ganze System (auch nicht bestrahlte Flächen) empfindlich gegenüber dem DLP-Verfahren, da durch den Eintrag der Energie die ganze Wanne (das Behältnis für das flüssige Harz)homogen gehärtet werden kann, ohne die ganze Wanne bestrahlen zu müssen. Dieser wichtige Aspekt wurde in der hier vorgestellten Erfindung beachtet und so verändert, dass dieses Problem außer Acht gelassen werden konnte.

Obwohl mit dem Produkt von Carbon elastische Zusammensetzungen mit einem geringen Anteil an Siloxan Gruppen entwickelt wurden, muss erwähnt werden, dass es sich hierbei um ein 2K-Material handelt, welches nach dem Druckprozess mit Wärme behandelt werden muss, um die beschriebenen Eigenschaften und ein fertiges Produkt zu erhalten.

Des Weiteren besteht ein Bedarf für transparente, biokompatible 1K-Materialien im DLP-Druck, welche ähnliche Eigenschaften wie klassisches Silikon, also elastische, physiologisch unbedenkliche und mechanisch belastbare Eigenschaften, aufweisen. Bekannte transparent elastische 3D-Druck Materialien können entweder nicht durch das klassische DLP-Verfahren gedruckt werden, sind nicht biokompatibel, weisen keine Transparenz auf und/oder können nicht in den Bereich der klassischen Silikone, aufgrund ihrer Zusammensetzung, eingeteilt werden.

Daher besteht auf dem Fachgebiet ein Bedarf für transparent elastische und biokompatible 3D-druckbare Materialien, welche einen hohen Anteil an Siloxan Gruppen aufweisen und mit dem klassischen DLP-Verfahren gedruckt werden können.

Im Stand der Technik gibt es verschiedene Materialien, die die einzelnen Eigenschaften zwar aufweisen, aber es gibt kein Material, welches alle Eigenschaften gleichzeitig zusammenfasst.

Das ist mit dieser Erfindung gelungen. Die Erfindung hat damit ein Material geschaffen, das alle Eigenschaften vereint.

Die Formulierung gemäß der Erfindung enthält sowohl Acrylat als auch Methacrylat funktionalisierte Gruppen und kann ausschließlich durch einen entsprechenden Photoinitiator oder eine Kombination aus mehreren Photoinitatoren im UV-Licht Bereich polymerisiert und somit gehärtet werden. Insbesondere die Wellenlängenbereiche von 350 bis 410 nm eignen sich dafür. Die Siloxanhaltigen (Meth)acrylate sollen dabei auch andere Funktionalitäten nämlich eine Urethangruppe enthalten. Diese Gruppe sorgt unter anderem durch intramolekulare Wechselwirkungen für mechanisch belastbare Eigenschaften.

Insbesondere stellt die Erfindung eine Zusammensetzung gemäß Anspruch 1 zur Verfügung, welche folgende Strukturen umfasst:
a) ein Monomer oder Oligomer mit der Strukturformel: 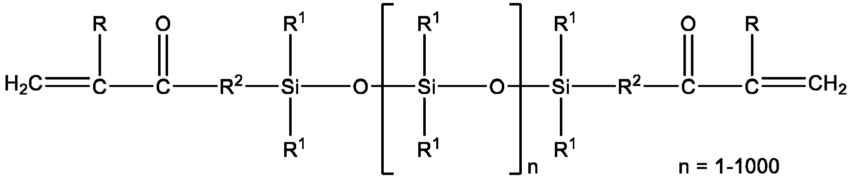 wobei R ein Wasserstoff oder ein C₁₋₂₀-Alkyl ist, das substituiert oder unsubstituiert sein kann oder eine ungesättigte radikalisch härtende Gruppe ist. R¹ ist ebenfalls ein C₁₋₂₀-Alkyl, welches substituiert oder unsubstituiert sein kann. R² beschreibt eine Urethangruppe, welche anschließend dargestellt werden soll: 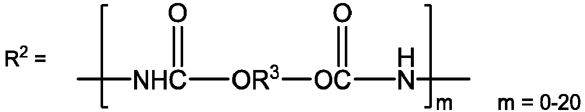 und wobei m minimal 1 ist,
   R³ stellt ein C₁₋₆-Alkyl dar, das substituiert oder unsubstituiert sein kann oder cyclisch gesättigt oder ungesättigt sein kann.
b) ein oder mehrere Monomer(e) mit der Strukturformel: 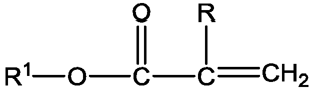 wobei R ein Wasserstoff oder ein C₁₋₂₀-Alkyl ist, das substituiert oder unsubstituiert sein kann oder eine ungesättigte radikalisch härtende Gruppe ist. R¹ ist ein cyclisches
   C₁₋₂₀-Alkyl, das substituiert oder unsubstituiert sein kann, cyclisch gesättigt oder ungesättigt sein kann oder aus zwei aneinander gekoppelten Cyclen oder voneinander unabhängigen Cyclen bestehen kann. Gemäß Anspruch 3 ein aliphatisches oder cycloaliphatisches Dimethacrylat mit einer Viskositöät von < 10 Pa·s.
c) ein Silikonöl mit der Strukturformel: 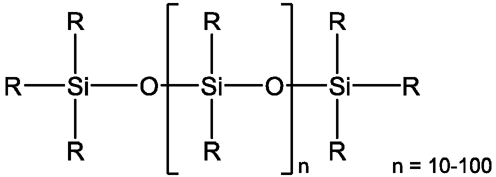 wobei R ein C₁₋₂₀-Alkyl ist, das substituiert oder unsubstituiert sein kann und cyclisch gesättigt oder ungesättigt sein kann.
d) eine pyrogene oder gefällte Kieselsäure, welche hydrophil oder hydrophob modifiziert sein kann.
e) ein oder eine Kombination von Photoinitiator(en) mit der Strukturformel: 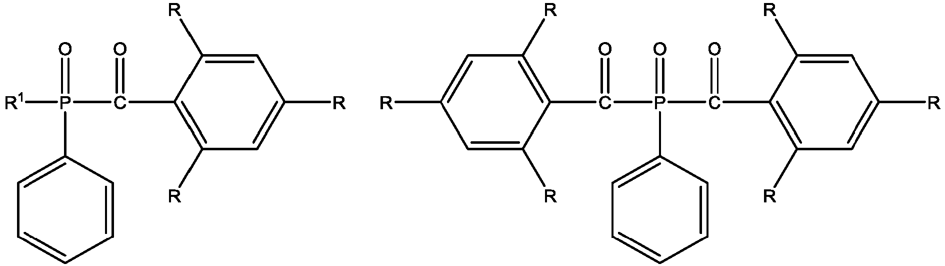 bei der R Wasserstoff oder ein C₁₋₆-Alkyl ist, das substituiert oder unsubstituiert sein kann oder cyclisch gesättigt und ungesättigt sein kann. R¹ ist eine Alkoxygruppe oder C₁₋₆-Alkyl Gruppe, welche substituiert oder unsubstituiert sein kann.

Lösung der vorliegenden Erfindung ist es, eine niedrigviskose, transparente und biokompatible Silikon-Harzformulierung für die Produktion von Medizinprodukten mittels konventioneller Stereolithographie zur Verfügung zu stellen, die sowohl die mechanischen, insbesondere eine Zugdehnung von > 100%, toxikologisch als auch die an die o.g. Verfahren gestellten Anforderungen erfüllt. In diesem Fall wurde gefunden, dass eine niedrigviskose, aus einem oder mehreren oligomeren Di(meth)acrylaten auf Basis von Silikonurethanen, das außerdem ein cyclisches (Meth)acrylat mit einer Viskosität von < 0,5 Pa·s, weiterhin ein Silikonöl mit einer Viskosität < 1 Pa·s, für die Technik der Stereolithographie eingesetzt werden kann und das bei der Härtung mittels Laser oder unter (UV-)Licht geeigneter Wellenlänge vorgehärtete Formkörper ergibt, die sich durch eine Grünfestigkeit auszeichnen, welche weitaus weicher und flexibler sind als die Endprodukte. Überraschenderweise wurde gefunden, dass durch Umstellung der Formulierung nicht nur elastische, sondern zunächst weiche, flexible, kunststoffähnliche Körper mit einem Silikonanteil von 50% und einer hervorragenden Mechanik, welche nach der Nacharbeitung erstarren, hergestellt werden können. Zudem konnten Zusammensetzungen hergestellt werden, welche schon als Grünkörper starre Strukturen ausgebildet haben, welche anschließend ebenfalls keine weichen Eigenschaften vorwiesen. Der Gegenstand der vorliegenden Erfindung ist dementsprechend eine niedrigviskose, strahlungshärtbare, biokompatible Harzformulierung, welche zunächst gelb aber auch transparent sein kann und nach dem Einsatz in der Stereolithographie, genauer dem DLP-Verfahren und anschließender Nacharbeitung transparente elastische Formkörper ergibt, welche folgende Zusammensetzung enthält:
a) 25-75 Gew.-% eines monomeren oder oligomeren Dimethacrylats auf Basis von Siloxanurethanen mit einer Viskosität von < 100 Pa·s;
b) 20-50 Gew.-% eines oder mehrerer monomer/oligomer cyclischer (Meth)acrylate mit einer Viskosität von < 0,5 Pa·s;
   gemäß Anspruch 3 bis zu 20 Gew.-% eines aliphatischen oder cycloaliphatischen Dimethacrylats mit einer Viskosität von < 10 Pa·s;
c) 1-25 Gew.-% Silikonöl mit einer Viskosität von < 1 Pa·s;
d) 0,5-20 Gew.-% Füllstoffe mit einer Partikelgröße <100 µm;
e) 0,1-5 Gew.-% eines oder einer Kombination mehrerer Photoinitiatoren, deren Absorption im Wellenlängenbereich des eingesetzten Laserstrahles bzw. der Bestrahlungsquelle liegt;

gemäß Anspruch 6 bis zu 0,5 Gew.-% eines oder mehrerer anaerober Inhibitoren;
gemäß Anspruch 7 bis zu 5 Gew.-% an Farbmitteln;
gemäß Anspruch 8 bis zu 5 Gew.-% an üblichen Additiven wie UV-Stabilisatoren, Haftvermittlern und Verlaufsadditiven,
wobei der Anteil aller Komponenten zusammen 100 Gew.-% beträgt.

Vorzugsweise enthält ein erfindungsgemäßes Gemisch:
a) 30-65 Gew.-% eines monomeren oder oligomeren Dimethacrylats auf Basis von Siloxanurethanen mit einer Viskosität von < 100 Pa·s;
b) 10-25 Gew.-% eines oder mehrerer monomer/oligomer cyclischer (Meth)acrylate mit einer Viskosität von < 0,5 Pa·s;
   gemäß Anspruch 3 bis zu 20 Gew.-% eines aliphatischen oder cycloaliphatischen Dimethacrylats mit einer Viskosität von < 10 Pa·s;
c) 5-25 Gew.-% Silikonöl mit einer Viskosität von < 1 Pa·s;
d) 3-10 Gew.-% Füllstoffe mit einer Partikelgröße < 100 µm;
e) 0,2-2 Gew.-% eines oder einer Kombination mehrerer Photoinitiatoren, deren Absorption im Wellenlängenbereich des eingesetzten Laserstrahles bzw. der Bestrahlungsquelle liegt;

gemäß Anspruch 6 bis zu 0,5 Gew.-% eines oder mehrerer anaerober Inhibitoren;
gemäß Anspruch 7 bis zu 5 Gew.-% an Farbmitteln;
gemäß Anspruch 8 bis zu 5 Gew.-% an üblichen Additiven wie UV-Stabilisatoren, Haftvermittlern und Verlaufsadditiven,
wobei der Anteil aller Komponenten zusammen 100 Gew.-% beträgt.

Als Verbindungen der Komponente (a) eignen sich beispielsweise funktionelle Oligomere aus einer Kombination von Silikon Urethan Acrylat Oligomeren, oder Silikon Urethan Methacrylat Oligomeren . Verwendet werden monomere oder oligomere Di(meth)acrylate auf Basis von Siloxanurethanen insbesondere eine Kombination aus Silikon Urethan Acrylat Oligomeren. Besonders bevorzugt sind dabei Kombination mit einem erhöhten Gewichtsanteil an Silikon innerhalb der Kombination aus Silikon Urethan Acrylat Oligomeren.

Die in der erfindungsgemäßen Formulierung als Komponente (b) eingesetzten Monomere oder Oligomere cyclischer (Meth)acrylate mit einer Viskosität von < 0,1 Pa·s sind kommerziell erhältlich bei der Firma Arkema. Bekannte Vertreter für diese Moleküle sind Isobornylacrylat und dessen Derivate, Trimethylcyclohexyl(meth)acrylate und dessen Derivate, Tert. Butylcyclohexyl(meth)acrylat und die zugehörigen Derivate, Cyclotrimethylpropanformal(meth)-acrylat und dessen Derivate, Isobornylcyclohexyl(meth)-acrylat und dessen Derivate. Vorzugsweise werden letztere verwendet mit einer Viskosität < 0,02 Pa·s.

Als Verbindung der Komponente gemäß Anspruch 3 können beispielsweise eingesetzt werden:
Urethan(meth)acrylate mit einer Funktionalität < 4, die dem Fachmann bekannt sind und in bekannter Weise hergestellt werden können, indem man beispielsweise ein hydroxylterminiertes Polyurethan mit Methacrylsäure zum entsprechendem Urethanmethacrylat umsetzt, oder indem man ein isocyanatterminiertes Präpolymer mit Hydroxymethacrylaten umsetzt. Entsprechende Verfahren sind z.B. aus EP 0579503 bekannt. Urethan(meth)acrylate sind auch im Handel erhältlich und werden beispielsweise unter der Bezeichnung PC-Cure^{®} von der Firma Piccadilly Chemicals, unter der Produktbezeichnung CN 1963 von der Firma Sartomer, unter der Bezeichnung Photomer von der Firma Cognis, unter der Bezeichnung Ebecryl von der Firma UCB und unter der Bezeichnung Genomer^{®} von der Firma Rahn vertrieben. Bevorzugt werden als Urethan(meth)acrylate solche eingesetzt, die n <4 funktionalisiert sind, Viskositäten < 15 Pa·s besitzen, ein Molekülgewicht < 2000 haben, und aus aliphatischen Edukten hergestellt worden sind. Insbesondere findet das aus HEMA und TMDI erhaltene Isomerengemisch 7,7,9- (bzw. 7,9,9-)Trimethyl-4,13-dioxo-3,14-dioxa-5,12-diazahexadecan-1,16-diol-dimethacrylat Anwendung.

Die in der Erfindung erwähnten Komponente (c) können beispielsweise lineare, nichtreaktive Silikonöle mit einer Siloxaneinheit von 10 bis 100 und Viskosität von <0,01 bis 0,1 Pa·s. Solche Produkte können von der Firma Momentive (Leverkusen) oder Wacker Chemie AG erworben werden.

### Beispiel 1:

| | |
|---|---|
| 50,6*(1-x/100) Gew.-% | Silikon Urethan Acrylat (Viskosität < 20 Pa s) |
| 36,1*(1-x/100) Gew.-% Acrylat | Trimethylcyclohexyl |
| 9,2*(1-x/100) Gew.-% 0,015 | (Viskosität < 0,01 Pa s) Silikonöl (Viskosität < Pa s) |
| 3,1*(1-x/100) Gew.-% 1,0 *(1-x/100) Gew.-% | pyrogene Kieselsäure Phenylbis(2,4,6-trimethylbenzoyl) phosphine oxide |

Farbe: klar transparent
Reissdehnung (%): 250 ± 8
Zugfestigkeit (MPa): 4,5 ± 0,1
Durometer: 70 Shore A

### Beispiel 2:

| | |
|---|---|
| 56,3*(1-x/100) Gew.-% | Silikon Urethan Acrylat (Viskosität < 20 Pa s) |
| 28,2*(1-x/100) Gew.-% (Viskosität | Isobornyl Acrylat < 0,015 Pa s) |
| 11,2*(1-x/100) Gew.-% 0,015 | Silikonöl (Viskosität < Pa s) |
| 3,3*(1-x/100) Gew.-% 1,0 *(1-x/100) Gew.-% | pyrogene Kieselsäure Phenylbis(2,4,6-trimethylbenzoyl) phosphine oxide |

Farbe: klar transparent
Reissdehnung (%): 201 ± 6
Zugfestigkeit (MPa): 3,8 ± 0,1
Durometer: 60 Shore A

### Beispiel 3:

| | |
|---|---|
| 52,1*(1-x/100) Gew.-% | Silikon Urethan Acrylat (Viskosität < 20 Pa s) |
| 32,8*(1-x/100) Gew.-% (Viskosität | Isobornyl Acrylat < 0,015 Pa s) |
| 11,0*(1-x/100) Gew.-% 0,015 | Silikonöl (Viskosität < Pa s) |
| 1,1*(1-x/100) Gew.-% 1,0 *(1-x/100) Gew.-% | pyrogene Kieselsäure Phenylbis(2,4,6-trimethylbenzoyl) phosphine oxide |

Farbe: klar transparent
Reissdehnung (%): 100 ± 5
Zugfestigkeit (MPa): 3,4 ± 0,2
Durometer: 50 Shore A

### Beispiel 4:

| | |
|---|---|
| 54,9*(1-x/100) Gew.-% | Silikon Urethan Acrylat (Viskosität < 20 Pa s) |
| 30,0*(1-x/100) Gew.-% | modifiziertes Isobornyl Acrylat (Viskosität < 0,015 Pa s) |
| 8,8*(1-x/100) Gew.-% Trimethylpropan-formal(meth)acrylat | cyclisches (Viskosität< 0,015 Pa s) |
| 4,0*(1-x/100) Gew.-% 0,015 | Silikonöl (Viskosität < Pa s) |
| 1,1*(1-x/100) Gew.-% 1,2 *(1-x/100) Gew.-% | pyrogene Kieselsäure Phenylbis(2,4,6-trimethylbenzoyl) phosphine oxide |

Farbe: klar transparent
Reissdehnung (%): 90 ± 5
Zugfestigkeit (MPa): 3,9 ± 0,2
Durometer: 60 Shore A

### Beispiel 5:

| | |
|---|---|
| 53,4*(1-x/100) Gew.-% | Silikon Urethan Acrylat (Viskosität < 20 Pa s) |
| 32,5*(1-x/100) Gew.-% | modifiziertes Isobornyl Acrylat (Viskosität < 0,015 Pa s) |
| 3,3*(1-x/100) Gew.-% Trimethylpropan-formal(meth)acrylat | cyclisches (Viskosität< 0,015 Pa s) |
| 3,5*(1-x/100) Gew.-% | 2-Ethyl-hexylacrylat (Viskosität< 0,015 Pa s) |
| 4,1*(1-x/100) Gew.-% 0,015 | Silikonöl (Viskosität < Pa s) |
| 2,0*(1-x/100) Gew.-% 1,2 *(1-x/100) Gew.-% | pyrogene Kieselsäure Phenylbis(2,4,6-trimethylbenzoyl) phosphine oxide |

Farbe: klar transparent
Reissdehnung (%): 105 ± 5
Zugfestigkeit (MPa): 3,1 ± 0,2
Durometer: 45 Shore A

### 3D druckbare Silikon im Vergleich:

| **Eigenschaften** | ***Carbon Silicone*** | ***Wacker AG*** | ***Keyence*** | ***Erfindung*** |
|---|---|---|---|---|
| **Farbe** | Grau opak | Farblos transparent | Weiß opak | Farblos transparent |
| **Reissdehnung** (%) | 330 | 200-800 | 160 | 100 |
| **Zugfestigkeit (MPa)** | 3.2 | 20-60 | 0.5-0.8 | 3.5 |
| **Durometer (Shore A)** | 35 | 20-60 | 35 & 65 | 45 |
| **Verfahren** | CLIP | Drop-on-Demand | Polyjet | DLP |
| **Anteil an Silikon** (%) | 30^{∗} | 100 | 70^{∗} | 65 |

| | | | | |
|---|---|---|---|---|
| ***geschätzt** | | | | |

Quellen:
Carbon: https://www.carbon3d.com/materials/silicone/
Wacker AG: https://www.aceo3d.com/silicones/
Keyence: https://eb-tec.de/wp-content/uploads/2017/09/Elastisches-Silikon-Druckmaterial.pdf

Ein wichtiger Unterschied der Erfindung zu den anderen 3D druckbaren Silikonen ist, dass sie ähnliche oder bessere Eigenschaften aufweisen und gleichzeitig mit dem gängigen DLP Verfahren gedruckt werden kann.

Bei der Komponente a) sind in einer bevorzugten Formulierung 50 bis 60 Gew.-% enthalten, weil hierdurch entsprechend gewünschte elastische Materialien erzeugt werden können. Grenzbereiche, bei denen gerade noch eine ausreichende elastische Materialqualität erzeugt wird, liegen bei 25 bis 75 %, vorzugsweise bei 35 bis 65 %.

Bei der Komponente b) deckt der angegebene Bereich von 20 bis 50 Gew.-% alle elastischen und möglicherweise interessanten Bereiche ab. 20 Gew.-% müssen enthalten sein, um die Viskosität der Gesamtzusammensetzung niedrig zu halten. Mehr als 50 % ergeben plastische Objekte und nicht mehr elastische Objekte.

Bezüglich der Komponente gemäß Anspruch 3 ist anzumerken, dass die Zugabe dieses Bestandteiles nicht zwingend ist. Es können aber durch Zugabe die mechanischen Eigenschaften verbessert werden. Nachteile können sich aber ergeben, weil die Objekte möglicherweise nicht mehr transparent sind oder die Viskositäten nicht niedrig genug sind.

Bezüglich der Komponente c) ist anzumerken, dass die Verwendung von Silikonöl essentiell ist. Es erniedrigt die Viskosität und verstärkt zudem die elastischen Eigenschaften im System. Die Zugabe im Bereich von 1 bis 25 Gew.-% ist variabel und zwar ohne Verfärbung des Systems.

Bezüglich der Komponente d) ist anzumerken, dass solche Füllstoffe ebenfalls das elastische Verhalten verstärken können. In einer zu großen Menge können sie aber zu einer Thixotropie führen, welche die Fließfähigkeit negativ beeinflusst. Bevorzugt ist ein Bereich von Füllstoffen von 0,5 bis 10 Gew.-%. Bezüglich der Komponente e) ist anzumerken, dass ein Photoinitiator oder eine Kombination von Photoinitiatoren unverzichtbar für das lichthärtende System ist. Die Konzentrationen können aber auf 0,1 bis 3 Gew.-% reduziert werden, ohne Nachteile für das Ergebnis.

Bezüglich der Formulierung gemäß Anspruch 6 ist anzumerken, dass die Inhibitoren dem System nicht separat hinzugefügt werden müssen, sondern sie sind in den Monomeren enthalten. Es sollte hierbei ein Gewichtsanteil von maximal 0,5 Gew.-% eingestellt werden.

Bezüglich der Komponente gemäß Anspruch 7 ist anzumerken, dass Farbmittel für die primäre Anwendung nicht erwünscht sind, die aber dennoch verwendet werden könnten.

Bezüglich der Komponente gemäß Anspruch 8 ist anzumerken, dass es nicht zwingend erforderlich ist, UV-Stabilisatoren oder Verlaufsadditive in der Finalformulierung beizufügen. Sie können aber eingesetzt werden.

## Patentansprüche

1. Mittels stereolithographischer Verfahren, nämlich Digital Light Processing-Verfahren, druckbares, transparentes, elastisches und biokompatibles 1K Silikon für die generative Fertigung, mindestens enthaltend:
a) 25-75 Gew.-% eines monomeren oder oligomeren Dimethacrylats auf Basis von Siloxanurethanen mit einer Viskosität von < 100 Pa·s,
b) 20-50 Gew.-% eines oder mehrerer monomer/oligomer cyclischer (Meth)acrylate mit einer Viskosität von < 0,5 Pa·s,
c) 1-25 Gew.-% Silikonöl mit einer Viskosität von <1 Pa·s,
d) 0,5-20 Gew.-% Füllstoffe mit einer Partikelgröße <100 µm,
e) 0,1-5 Gew.-% eines oder einer Kombination mehrerer Photoinitiatoren, deren Absorption im Wellenlängenbereich des eingesetzten Laserstrahles bzw. der Bestrahlungsquelle liegt.

2. Formulierung nach Anspruch 1, **dadurch gekennzeichnet, dass** 35 bis 65 Gew.-% der Komponente a) enthalten sind.

3. Formulierung nach Anspruch 1 oder 2, **dadurch gekennzeichnet, dass** bis zu 20 Gew.-% eines aliphatischen oder cycloaliphatischen Dimethacrylats mit einer Viskosität von < 10 Pa·s enthalten sind.

4. Formulierung nach einem der Ansprüche 1 bis 3, **dadurch gekennzeichnet, dass** 0,5 - 10 Gew.-% der Komponente d) enthalten sind.

5. Formulierung nach einem der Ansprüche 1 bis 4, **dadurch gekennzeichnet, dass** 0,1 bis 3 Gew.-% der Komponente e) enthalten sind.

6. Formulierung nach einem der Ansprüche 1 bis 5, **dadurch gekennzeichnet, dass** bis zu 0,5 Gew.-% eines oder mehrerer anaerober Inhibitoren enthalten sind.

7. Formulierung nach einem der Ansprüche 1 bis 6, **dadurch gekennzeichnet, dass** Farbmittel bis zu 5 Gew.-% enthalten sind.

8. Formulierung nach einem der Ansprüche 1 bis 7, **dadurch gekennzeichnet, dass** bis zu 5 Gew.-% Additive, UV-Stabilisatoren, Haftvermittler und/oder Verlaufsadditive enthalten sind.

9. Formulierung nach einem der Ansprüche 1 bis 8, wobei das Gemisch enthält:
a) 25-75 Gew.-% eines monomeren oder oligomeren Dimethacrylats auf Basis von n-fach funktionalisiertem Siloxanurethanen mit einem Siloxangrad von n = 1-1000 und einem Urethangrad m = 0-20,
20-50 Gew.-% eines oder mehrerer monomer/oligomer cyclischer Methacrylate mit
einer Funktionalität von n < 2 und einer Viskosität von < 0,1 Pa·s und einem Molekülgewicht < 250,
c) 2-20 Gew.-% eines aliphatischen oder cycloaliphatischen Dimethacrylats mit einer Viskosität von < 10 Pa·s,
d) 2-25 Gew.-% Silikonöl mit einer Viskosität von < 1 Pa·s,
e) 2-20 Gew.-% Füllstoffe mit einer Partikelgröße < 100 µm,
f) 0,5-5 Gew.-% eines oder einer Kombination mehrerer Photoinitiatoren, deren Absorption im Wellenlängenbereich des eingesetzten Laserstrahles bzw. der Bestrahlungsquelle liegt.

10. Formulierung nach Anspruch 9, **dadurch gekennzeichnet, dass** bis zu 0,5 Gew.-% eines oder mehrerer anaerober Inhibitoren, auch in Verbindung mit in der Stereolithographie bekannten aeroben Inhibitoren enthalten sind.

11. Verfahren zur Herstellung von medizinischen Produkten und elastischen medizinischen Produkten, **dadurch gekennzeichnet, dass** die Produkte aus einer Formulierung gemäß einem der Ansprüche 1 bis 10 mittels eines lichthärtenden Verfahrens, nämlich dem Digital Light Processing-Verfahren, eines Licht härtenden generativen Verfahrens, hergestellt werden.

12. Verfahren nach Anspruch 11, **dadurch gekennzeichnet, dass** in der zu härtenden Formulierung oder Zusammensetzung eine Stützstruktur für den zu erstellenden Formkörper angeordnet wird.

13. Verwendung einer Formulierung nach einem der Ansprüche 1 bis 10 zur Herstellung von medizinischen Produkten, insbesondere von elastischen Produkten, **dadurch gekennzeichnet, dass** das Licht härtende generative Verfahren, nämlich Digital Light Processing-Verfahren, mit einer Strahlen härtbaren Zusammensetzung von Monomeren und/oder Prepolymeren erfolgt, wobei optional in der zu härtenden Zusammensetzung eine Stützstruktur für den zu erstellenden Formkörper vorgesehen ist.

## Claims

1. A transparent, elastic, and biocompatible 1-component silicone imprintable by means of a stereolithographic process, namely a Digital Light Processing method, for additive manufacturing, at least comprising:
a) 25-75 % by weight of a monomeric or oligomeric dimethacrylate based on siloxane urethanes having a viscosity of < 100 Pa·s,
b) 20-50 % by weight of one or more monomeric/oligomeric cyclic methacrylates having a viscosity of < 0.5 Pa·s,
c) 1-25 % by weight of silicone oil having a viscosity of < 1 Pa·s,
d) 0.5-20 % by weight of fillers having a particle size of < 100 µm,
e) 0.1-5 % by weight of one or a combination of multiple photoinitiators, the absorption of which lies in the wavelength range of the employed laser beam or of the irradiation source, respectively.

2. The formulation of claim 1, **characterized in that** 35 to 65 % by weight of the component a) are included.

3. The formulation of claim 1 or 2, **characterized in that** up to 20 % by weight of an aliphatic or cycloaliphatic dimethacrylate having a viscosity of < 10 Pa·s are included.

4. The formulation of one of claims 1 to 3, **characterized in that** 0.5 - 10 % by weight of the component d) are included.

5. The formulation of one of claims 1 to 4, **characterized in that** 0.1 to 3 % by weight of the component e) are included.

6. The formulation of one of claims 1 to 5, **characterized in that** up to 0.5 % by weight of one or more anaerobic inhibitors are included.

7. The formulation of one of claims 1 to 6, **characterized in that** up to 5 % by weight of coloring agents are included.

8. The formulation of one of claims 1 to 7, **characterized in that** up to 5 % by weight of additives, UV stabilizers, adhesion promoters, and/or leveling additives are included.

9. The formulation of one of claims 1 to 8, the mixture comprising:
a) 25-75 % by weight of a monomeric or oligomeric dimethacrylate based on n-fold functionalized siloxane urethanes having a siloxane degree of n = 1-1000 and a urethane degree of m = 0-20,
b) 20-50 % by weight of one or more monomeric/oligomeric cyclic methacrylates having a functionality of n < 2 and a viscosity of < 0.1 Pa·s and a molecular weight < 250,
c) 2-20 % by weight of an aliphatic or cycloaliphatic dimethacrylate having a viscosity of < 10 Pa·s,
d) 2-25 % by weight of silicone oil having a viscosity of < 1 Pa·s,
e) 2-20 % by weight of fillers having a particle size of < 100 µm,
f) 0.5-5 % by weight of one or a combination of multiple photoinitiators, the absorption of which lies in the wavelength range of the employed laser beam or of the irradiation source, respectively.

10. The formulation of claim 9, **characterized in that** up to 0.5 % by weight of one or more anaerobic inhibitors, also in connection with aerobic inhibitors known in the stereolithography are included.

11. A method for manufacturing medical products and elastic medical products, **characterized in that** the products are manufactured from a formulation of one of claims 1 to 10 by means of a photocuring process, namely the Digital Light Processing method, of a photocuring additive process.

12. The method of claim 11, **characterized in that** in the formulation or composition to be cured, a support structure for the shaped body to be generated is disposed.

13. Use of a formulation of one of claims 1 to 10 for manufacturing medical products, in particular elastic products, **characterized in that** the photocuring additive process, namely the Digital Light Processing method, occurs with a radiation-curable composition of monomers and/or prepolymers, optionally, in the composition to be cured, a support structure for the shaped body to be generated being provided.

## Revendications

1. Silicone monocomponsante transparente, élastique et biocompatible et imprimable au moyen d'un procédé stéréolithographique, c'est-à-dire un procédé Digital Light Processing, pour la fabrication additive, au moins comprenant:
a) 25-75 % en poids d'un diméthacrylate monomérique ou oligomérique sur la base de siloxane uréthanes avec une viscosité de < 100 Pa·s,
b) 20-50 % en poids d'un ou de plusieurs méthacrylates monomériques/oligomériques cycliques avec une viscosité de < 0,5 Pa·s,
c) 1-25 % en poids d'huile de silicone avec une viscosité de < 1 Pa·s,
d) 0,5-20 % en poids de charges avec une grandeur de particule de < 100 µm,
e) 0,1-5 % en poids d'un ou d'une combinaison de photoinitiateurs multiples, l'absorption desquels a lieu dans la gamme des longueurs d'onde du faisceau laser utilisé ou de la source d'irradiation, respectivement.

2. Formulation selon la revendication 1, **caractérisée en ce que** de 35 à 65 % en poids du composant a) sont compris.

3. Formulation selon la revendication 1 ou 2, **caractérisée en ce que** jusqu'à 20 % en poids d'un diméthacrylate aliphatique ou cycloaliphatique avec une viscosité de < 10 Pa·s sont compris.

4. Formulation selon une des revendications 1 à 3, **caractérisée en ce que** de 0,5 - 10 % en poids du composant d) sont compris.

5. Formulation selon une des revendications 1 à 4, **caractérisée en ce que** de 0,1 à 3 % en poids du composant e) sont compris.

6. Formulation selon une des revendications 1 à 5, **caractérisée en ce que** jusqu'à 0,5 % en poids d'un ou de plusieurs inhibiteurs anaérobiques sont compris.

7. Formulation selon une des revendications 1 à 6, **caractérisée en ce que** jusqu'à 5 % en poids de colorants sont compris.

8. Formulation selon une des revendications 1 à 7, **caractérisée en ce que** jusqu'à 5 % en poids d'additifs, de stabilisants UV, de promoteurs d'adhésion, et/ou d'additifs promoteurs d'écoulement sont compris.

9. Formulation selon une des revendications 1 à 8, le mélange comprenant:
a) 25-75 % en poids d'un diméthacrylate monomérique ou oligomérique sur la base de siloxane uréthanes n-fois fonctionnalisés avec un degré de siloxane de n = 1-1000 et un degré d'uréthane de m = 0-20,
b) 20-50 % en poids d'un ou de plusieurs méthacrylates monomériques/oligomériques cycliques avec une fonctionnalité de n < 2 et une viscosité de < 0,1 Pa·s et un poids moléculaire de < 250,
c) 2-20 % en poids d'un diméthacrylate aliphatique ou cycloaliphatique avec une viscosité de < 10 Pa·s,
d) 2-25 % en poids d'huile de silicone avec une viscosité de < 1 Pa·s,
e) 2-20 % en poids de charges avec une grandeur de particule de < 100 µm,
f) 0,5-5 % en poids d'un ou d'une combinaison de photoinitiateurs multiples, l'absorption desquels a lieu dans la gamme des longueurs d'onde du faisceau laser utilisé ou de la source d'irradiation, respectivement.

10. Formulation selon la revendication 9, **caractérisée en ce que** jusqu'à 0,5 % en poids d'un ou de plusieurs inhibiteurs anaérobiques, aussi en connexion avec des inhibiteurs aérobiques connus dans la stéréolithogra-phie sont compris.

11. Procédé de fabrication de produits médicaux et de produits médicaux élastiques, **caractérisé en ce que** les produits sont fabriqués à partir d'une formulation selon une des revendications 1 à 10 au moyen d'un procédé de durcissement à la lumière, c'est-à-dire du procédé Digital Light Processing, d'un procédé additif de durcissement à la lumière.

12. Procédé selon la revendication 11, **caractérisé en ce que** dans la formulation ou composition à être durcie, une structure de support pour la pièce à être générée est disposée.

13. Utilisation d'une formulation selon une des revendications 1 à 10 pour fabriquer des produits médicaux, en particulier des produits élastiques, **caractérisée en ce que** le procédé additif de durcissement à la lumière, c'est-à-dire le procédé Digital Light Processing, a lieu avec une composition durcissable à la radiation de monomères et/ou prépolymères, optionnellement, dans la composition à être durcie, une structure de support pour la pièce à être générée étant prévue.
